# EUROPEAN PATENT SPECIFICATION

(11) **EP 1 786 821 B1**
(45) Date of publication and mention of the grant of the patent: **16.09.2009**
(21) Application number: 05773570.6
(22) Date of filing: 19.07.2005
(51) Int. Cl.: G06F 17/30

(54) **DYNAMIC KNOWLEDGE-BASED NETWORKING SYSTEM AND METHOD**
DYNAMISCHES INFORMATIONSGESTÜTZTES NETZWERKSYSTEM UND VERFAHREN
SYSTEME ET PROCEDE DE RESEAUTAGE DYNAMIQUE A BASE DE CONNAISSANCES

(30) Priority: 19.07.2004 US 589355 P
(43) Date of publication of application: 23.05.2007
(73) Proprietor: Intercasting Corporation, Cardiff CA 92007 (US)
(72) Inventor: CONAHAN, Shawn, 1754 San Diego CA 92101 (US); OIEN, Derrick, Cardiff CA 92007 (US); DEMAS, Tom, Reseda CA 91335 (US)
(74) Representative: McCann, Heather Alison
(86) International application number: PCT/US2005/025972
(87) International publication number: WO 2006/012473

(56) References cited:
- EP-A- 1 176 840
- WO-A-02/099597
- WO-A-20/04046875
- US-A1- 2003 028 525
- US-A1- 2003 055 983
- US-A1- 2003 093 405
- US-B1- 6 434 599

## Description

The present application claims priority under 35 U.S.C. §119 to U.S. Provisional Application Serial Number 60/589,355, filed July 19, 2004, entitled DYNAMIC KNOWLEDGE-BASED NETWORKING SYSTEM AND METHOD.

### BACKGROUND

Mobile communication devices, commonly called "mobile devices," "cellular phone," "handsets" or "personal digital assistants," increasingly offer a wider array of network communication capabilities. For instance, many mobile devices can connect to the Internet to access Web content. A user can even search for desired Web content using a modified browser interface tailored for mobile applications. However, mobile devices have a number of characteristics that make search tools, particularly Internet search tools, ineffective.

Characteristics of mobile devices that make searches ineffective include size and form factor limitations, particularly in the user interface (UI) of the mobile device. The shortage of space available for display and presentation makes returning a large list of results or options a difficult and unpleasant user experience. Further, the data found on a website does not lend itself well to satisfactory presentation on a mobile device UI, and pulling up webpages based on the list of results or options is usually difficult.

Another issue for search on a mobile device is relevance. A keyword search performed on a search site such as Google, for example, will provide all of the available results found around the entered keywords. These results do not take into account mobile context, such as a time-sensitivity of a request, a user's location, or more importantly, the subjective information or knowledge about a searched place or object that may be possessed by other persons familiar with the time or location context of the mobile device user.

Existing search and directory systems employ a number of different, generally web-based methods for acquiring information. One popular method for acquiring information is submitting a keyword query for comparison with a database of information to find matches, such as can be found in search engines like Yahoo or Google, although their techniques vary considerably. A related method can be used with a natural language search engine, such as Ask Jeeves, in which a user submits to the engine a natural language question, which is then parsed into a number of textual elements to identify a sequence or combination of words that can be matched against a database to answer the question.

Contextual search is another method used to find information. An example of contextual search is the result obtained from a commerce site such as Amazon in response to a user query (i.e., a title of a book that the user is looking to purchase). The commerce site can provide information about a number of other items that are somehow contextually related to the item being sought or purchased (i.e., titles of other books bought by users that also purchased the sought-after book). In this way, a user gets their asked-for search results, much like a keyword search, but also receives derivative information, such as other person's purchases or interests.

Another search method, particularly appropriate for mobile devices, is a location based search. In this method, a user provides their location to their mobile device or to a website via the mobile device, or their location is determined by a device similar to a Global Positioning System (GPS) device. Once the location is determined, the user can query the system to determine keywords of interest based on objects that have a relationship to that location. Examples of this method include Citysearch on the web and WaveMarket on a mobile device.

By way of an example, a user who is presently visiting Austin Texas may be interested in listening to a live performance of "emo" music, a certain style of music that is loosely associated with and sometimes classified as "rock" music, but which has only a small group of ardent fans with knowledge about the emo scene in Austin. The user may be seeking a live emo music venue. Conventional search methods for live music venues may yield a result of a very large number of live rock music venues in Austin or beyond. Further, this result can be arranged in a number of ways that are more distracting than helpful. Thus, conventional search methods typically do not consider timeliness (i.e., the user is seeking information that relates to the present), the location of the user (i.e., some results may not even apply to Austin, TX), nor the knowledge that others possess, such as the small yet passionate fan base.

The collective knowledge of a group of people that are related in some, and often many, ways is known as "tribal knowledge," and can often be the most reliable information about a subject. For example, the group of people may be users of a particular communications network that can be accessed via a mobile device. Further, subgroups of these users may be related by various interests, opinions, or knowledge about one or more subjects. Unfortunately, conventional search tools for mobile devices have no way of searching and identifying any of these user's in an efficient or reliable manner.
WO 2004/046875 discloses a system of dynamically informing a user of a network of other network users, wherein an online context of the user is dynamically determined. Other users within the online context of the user are identified and trait information is stored that is rotated essentially only to the user or to the users in a users store associated with the online context.
US 2003/0055983 discloses at least one method for providing virtual context such as a method for providing a journal, the method including creating a journal entry that is virtually affixed to a location of interest. Furthermore, the journal entry can be presented to a selected person when the selected person is within the vicinity of the location of interest.
US 2003/0028525 discloses a system which routes incoming electronic messages in a forum to subject matter experts for resolution. The subject matter experts are associated with their subject matter expertise through a database. A profile containing associated key words is created and may be used to identify a subject matter expert. A profiling platform matches key phrases that are entered with the information contained in the profiles.

### SUMMARY

A system and method is presented for creating a dynamic network of knowledge links for mobile devices for communicating with network members to obtain derivative or subjective information. One method includes creating an index in the same way that people use phone numbers for phones, web addresses for PCs, etc., whereby an index is generated to include a list of people with specific knowledge about specific things.

As opposed to conventional mobile search methods, in which a directory search is initiated by a user for the purpose of obtaining information such as a collection of relevant links, from a database of information or in the case of Google, relevant direct hits of a collection or links, the subject matter described herein utilizes other users to provide such information. While the methods used to get to the information are different, all of them involve utilizing some algorithm for finding the information and then presenting the direct result to the user. In addition, the subject matter described herein, in some variations, assumes that the most relevant and up to date information is contained within the knowledge of users and in some variations, and enables users to connect with each other to obtain that information. Because the subject matter described herein, in some variations, relies on users to provide the search results, the user seeking information may not use keywords that describe the question they are seeking to answer or the information they are trying to find, rather they would be using keywords to describe people or concepts they think would possess the information they are seeking.

According to one aspect, a networking system for mobile device users includes a database that is accessible via a communications network. The database stores information related to one or more users of the communications network. The networking system further includes an information engine configured to receive a query from a mobile device via the communications network. The information engine is further configured to determine a context of the query, compare the query and/or context with the information in the database to generate a contextual result, identify one or more users associated with the contextual result, and generate a directory of identifiers representing the identified one or more users.

According to another aspect, a method that connects various users of a communications network is provided. The method may be in the form of application software running on a computer such as a server, and executing a number of modules configured for particular functions to manipulate information stored in a database. Alternatively, the method can be implemented on a mobile device, in software, in hardware such as an application specific integrated circuit (ASIC), in firmware, or a combination thereof.

In some embodiments, a method includes the functional steps of receiving a query from a mobile device via a communications network, and determining a context of the query. The method can further include comparing the query and/or context with information in a database that is accessible via the communications network to generate a contextual result, where the database stores information related to one or more users of the communications network. The method further includes identifying one or more users associated with the contextual result, and generating a directory of identifiers representing the identified one or more users.

In a particular aspect, the systems and methods described herein are configured to generate a directory of one or more users of a communications network that may have knowledge about the subject of a query, rather than simply a directory limited only to the subject itself. Accordingly, in other embodiments a method can include receiving, from a mobile device via the communications network, a query of a database about one or more things within a context. The method can further include generating, based at least in part on the context, a directory of users of the communications network who have provided information to the database that represents a knowledge about the one or more things.

Articles of manufacture, tangibly embodied on a machine-readable medium, are also described. Such articles of manufacture may include executable instructions that cause a machine to conduct one or more of the method acts described herein.

Similarly, systems are also described that may include a processor and a memory coupled to the processor. The memory may encode one or more programs that cause the processor to perform one or more of the method acts described herein.

The details of one or more variations are set forth in the accompanying drawings and the description below. Other features and advantages of the subject matter described herein will be apparent from the description and drawings, and from the claims. the object of the invention is achieved with the features of the claims.

### BRIEF DESCRIPTION OF THE DRAWINGS

These and other aspects will now be described in detail with reference to the following drawings.

FIG. 1 is a block diagram of a dynamic group-based knowledge and information system.

FIG. 2 is a functional block diagram of a mobile information application.

FIG. 3 is a functional flow diagram of a dynamic knowledge-based method for search and directory using a mobile device.

FIGS. 4A-C show various elements of a functional application layer of the mobile information application.

Like reference symbols in the various drawings indicate like elements.

### DETAILED DESCRIPTION

The following describes systems, apparatuses, articles of manufacture, and methods for group-based knowledge and information search and retrieval via mobile devices. The mobile devices are network-connected and "always-on," in that they can be accessed by a user at any time and at any place. In some embodiments, systems and methods leverage one or more interrelationships of a group of users based on predetermined criteria to provide information that may not be known to other users. Accordingly, a directory of people can be created for the purpose of gaining derivative and subjective information more effectively than traditional data directory-based search techniques.

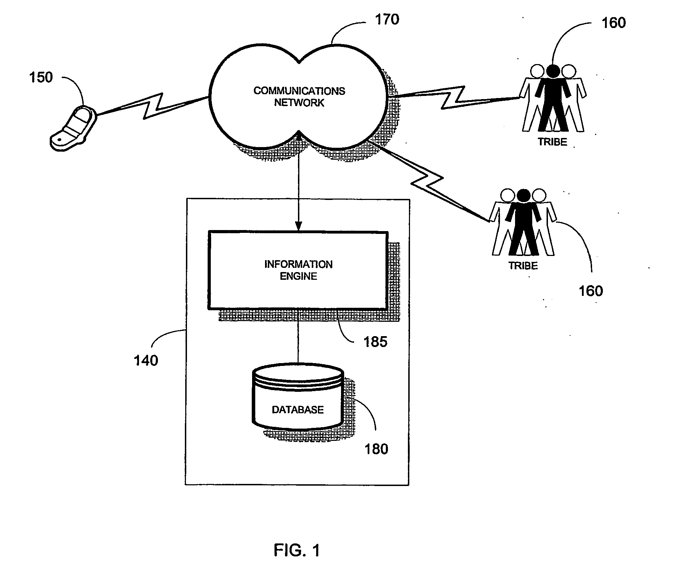

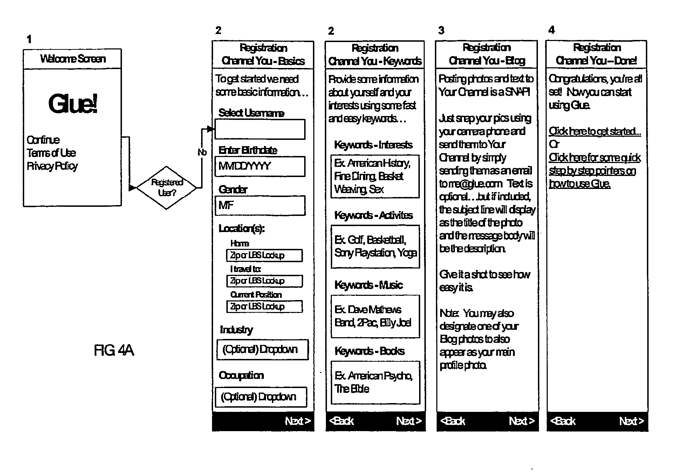

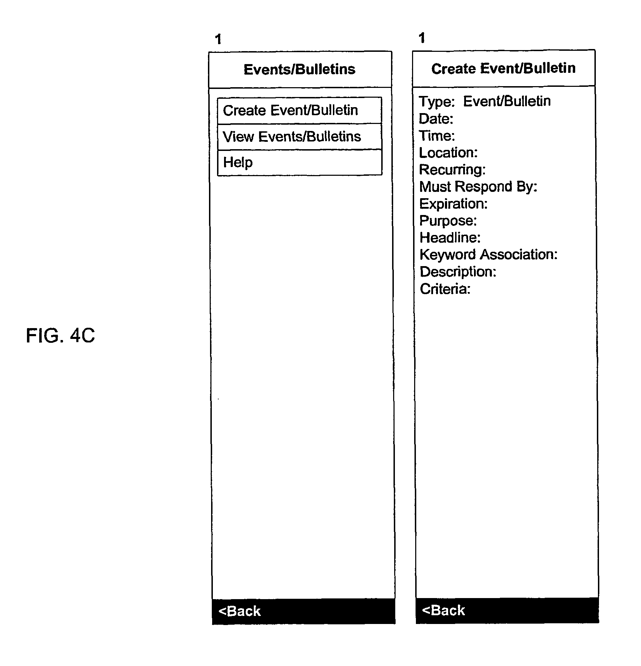

FIG. 1 includes a block diagram of a networking system 140 for networking a group of mobile device users based on dynamic group-based knowledge. In some variations, the networking system 140 includes a database 180 that is accessible via a communications network 170. The database 180 stores information related to one or more users 160 of the communications network 170, a contextually-related group of which is called a "tribe." The information can include text inputs, graphics, images, weblogs, or other information obtained through interactive screens or webpages. FIG. 4A illustrates a logic flow of an information gathering module. The information can be provided by the users via mobile device, over wireless data or voice channels, or via other network-enabled device such as a computer, PDA, etc.

The system 140 further includes an information engine 185 configured to receive a query from a mobile device 150 via the communications network 170 and determine a context of the query. The information engine 185 compares the query and/or context with the information in the database 180 to generate a contextual result, and to identify one or more users associated with the contextual result. The information engine 185 can generate a directory of identifiers representing the identified one or more users 160. Accordingly, based on a relevancy between the information related to the one or more users 160 and the query, a user using a mobile device 150 may contact the identified one or more users.

The system 140 enables a user using a mobile device 150 to access knowledge and information contained in one or more groups of users, called "tribes" 160. A user is associated with a tribe based on some knowledge that corresponds to a contextual relation with other users. For instance, one tribe 160 may be formed of users who have a common interest in emo music, but more specifically a live venue for emo music, and even more specifically a live venue for emo music in Austin, TX. The user 150 may or may not initially be a member of a tribe 160.

The mobile device 150 is any device capable of communicating with the communications network 170 which can include, without limitation, networks such as a wireless data and/or voice network, the Internet or world wide web, or other network The mobile device 150 may also be capable of multiple integrated communication with technologies such as short message service (SMS) messaging, electronic mail, mobile web connectivity, and one or more various voice communication technologies such as code division multiple access (CDMA) or its variants, or the global system for mobile (GSM) communications standard.

The mobile device 150 is used to generate and send information requests, in the form of keyword search queries or other types of requests, to the database 180 via the communications network 170. The requests are evaluated against the database 180 of information about one or more groups of users 160 or tribes. The tribes can be composed of overlapping groups of users 160. A result, which may be in the form of a directory, that represents one or more target users from at least one tribe is generated and returned to the mobile device 150 in response to the query. A user of the mobile device 150 can then use one of various forms of communication to contact one or more of the target users from the result for obtaining further specific information regarding the query.

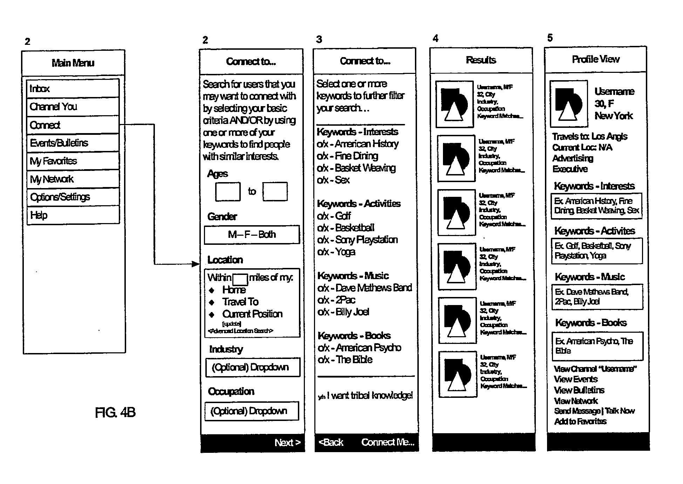

In some variations the system 140 includes an application that connects an associated network of users by matching the query with key words and metadata in user profiles and other information repositories (including blogs and media collections) stored in the database 180 for the purpose of finding information directly from another user in the network. In the example cited above, a search for bands in Austin would result in a list of other users who have provided information in their profile or other repository to the database 180 that represents their specific knowledge of music or band names related to the search. The user querying the system 140 can then look at a brief summary of the user's profiles, as shown in FIG. 4B, to identify those users who may possess the specific knowledge sought. Once the user identifies a target user they would like to query, they can send a message via a messaging infrastructure or through email or SMS to the respective target users. The target users would receive the message and respond if they have information regarding the query.

The networking system 140 can include a server running an application. The application can be distributed in part to the mobile device 150, either by downloading the application from a website, downloading the application directly from the carrier network through an application deck provided by the carrier, or by accessing the application via a web-like gateway using XHTML. The application can either run in an operating environment such as Java or Brew, or executed via a browser client on the mobile device 150 using XHTML or the like. The networking system 140 leverages the feature that users always or nearly always carry their mobile devices. Rather than create a directory of information about things, the system 140 generates a directory of people with knowledge about things.

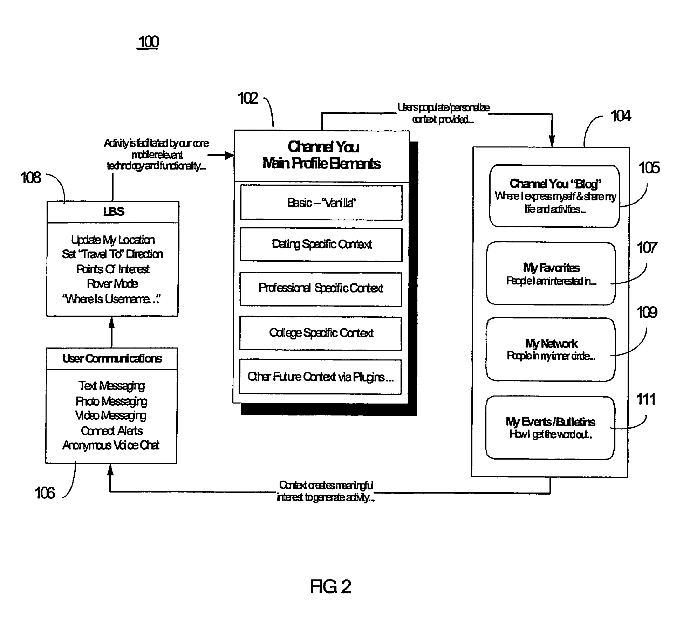

FIG. 2 is a functional block diagram of an application 100 running in the information engine 185 or distributed across devices communicating via the communication network 170. The application 100 includes a main profile 102 having various descriptive elements that describe a user, including location, age, preferences, etc. Other examples of descriptive elements are shown in FIG. 4A. The application 100 uses information of the main profile 102 to populate/personalize contextual elements 104 that link the information together based on one or more contextual arrangements for a more complete description of the user. For example, the contextual elements 104 can include a weblog 105, a "my favorites" element 107, a "my network" element 109 that lists people considered to be related in some way, and an events/bulletin element 111. These contextual elements 140 are merely exemplary, and are not to be construed as limiting the scope or number of possible contextual elements 104. Other contextual elements and element types are possible within the scope of this description.

The application 100 also includes management of and connectivity to one or more user communications 106, such as text messaging, photo messaging, video messaging, alerts, anonymous voice chat, or other communications. The application 100 also incorporates a location-based service 108, in which contextual elements are processed and updated according to a location of a mobile device being used by a user, for more temporally and contextually relevant information.

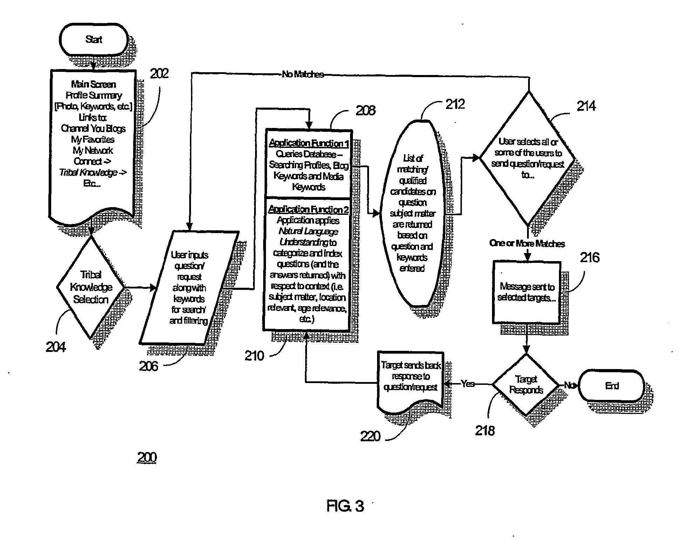

FIG. 3 is a functional flow diagram of a method 200 for dynamic, knowledge-based search and directory generation using a mobile device. At block 202, a mobile subscriber is provided a main screen, including objects such as a profile summary, a photo, and keywords, etc., for search and filtering. The main screen also includes links to various selectable web-based entities, such as weblogs ("blogs"), favorite links, links associated with a predefined network of users, and links to "tribal knowledge" web content.

A tribal knowledge selection, at block 204, is a link to data representing knowledge of a specifically-defined group of users with common interests or who have represented having particular predefined knowledge. Accordingly, the group of users constitutes an entity akin to a "tribe" and collectively provides the most relevant, directed results to search queries from any other user. Block 204 can be referred to generally as "Hook Me Up."

A user who selects the tribal knowledge selection is presented with a user interface at block 206, such as a GUI display, that prompts the user to enter keywords. The keywords may comprise text, although may also be in the form of images, graphics, etc. In some variations, a script instructs users to enter keywords that would be descriptive of users in the "tribe," or their own experiences, to "*Hook Up*" the user with an information source that, based on information provided by other users, is likely to have relevant information. The user then enters the keywords and the mobile information application, at blocks 208 or 210 or both, queries a database for relevant matches and returns a list of targeted users and their associated profiles (as shown in FIG. 4B) at block 212. The requesting user can browse the profiles to find one or more target users that are most likely to have the sought-after information.

Once the relevant target users are selected at block 214, the requesting user at block 216 can communicate to them (i.e. send a question or comment) using one of several forms of communication integrated into the system, such as SMS, email or other form communication mechanism. The requesting user then waits for the target(s) to respond to his or her request for information, at block 218. If no target user responds, the method 200 ends or begins again at block 214 or 216. If at least one target user responds with information, at block 220 the requesting user receives the information. The requesting user can end the method 200 or again execute the mobile information application for more information, at block 210. In some variations, the application can apply a natural language understanding program to categorize and index the query, as well as the returned answers, in a context or to generate a contextual result. The context can be related to subject matter, location relevance, age relevance, etc. The contextual result helps classify all of the information to better filter relevant information for the requesting user.

Example applications of the method 200 include generating referrals and generating a database of opinions/reviews. In an application for generating referrals, for example, a user may looking for a particular kind of club, for a particular type of band, for a live music venue, for a rave, for a party, for a service provider, for a restaurant, for a movie theater, for a store, or for a place to go, etc. The user is looking for a referral directory of only those users who are relevant to the same context-based subculture, as based on the query or request.

An application for generating opinions or reviews can be implemented as follows: A user asks the tribe, or subset thereof, (i.e. target users) "What do you think of a specific restaurant?" or "What did you think of this movie?" The target users can reply to the requesting user with an opinion or a review, which is then accumulated in the database to form information for later queries, as well as real-time information relevant to the requesting user.

FIGS. 4A-4C show various elements of a functional application layer of the mobile information application. The application layer is represented by various frames that depict user interface fields for receiving or displaying information to a user. It should be understood that a mobile information application is not necessarily limited to the exact number and types of frames depicted in FIGS. 4A-4C.

Various implementations of the subject matter described herein may be realized in digital electronic circuitry, integrated circuitry, specially designed ASICs (application specific integrated circuits), computer hardware, firmware, software, and/or combinations thereof. These various implementations may include implementation in one or more computer programs that are executable and/or interpretable on a programmable system including at least one programmable processor, which may be special or general purpose, coupled to receive data and instructions from, and to transmit data and instructions to, a storage system, at least one input device, and at least one output device.

These computer programs (also known as programs, software, software applications or code) include machine instructions for a programmable processor, and may be implemented in a high-level procedural and/or object-oriented programming language, and/or in assembly/machine language. As used herein, the term "machine-readable medium" refers to any computer program product, apparatus and/or device (e.g., magnetic discs, optical disks, memory, Programmable Logic Devices (PLDs)) used to provide machine instructions and/or data to a programmable processor, including a machine-readable medium that receives machine instructions as a machine-readable signal. The term "machine-readable signal" refers to any signal used to provide machine instructions and/or data to a programmable processor.

To provide for interaction with a user, the subject matter described herein may be implemented on a computer having a display device (e.g., a CRT (cathode ray tube) or LCD (liquid crystal display) monitor) for displaying information to the user and a keyboard and a pointing device (e.g., a mouse or a trackball) by which the user may provide input to the computer. Other kinds of devices may be used to provide for interaction with a user as well; for example, feedback provided to the user may be any form of sensory feedback (e.g., visual feedback, auditory feedback, or tactile feedback); and input from the user may be received in any form, including acoustic, speech, or tactile input.

The subject matter described herein may be implemented in a computing system that includes a back-end component (e.g., as a data server), or that includes a middleware component (e.g., an application server), or that includes a front-end component (e.g., a client computer having a graphical user interface or a Web browser through which a user may interact with an implementation of the subject matter described herein), or any combination of such back-end, middleware, or front-end components. The components of the system may be interconnected by any form or medium of digital data communication (e.g., a communication network). Examples of communication networks include a local area network ("LAN"), a wide area network ("WAN"), and the Internet.

The computing system may include clients and servers. A client and server are generally remote from each other and typically interact through a communication network. The relationship of client and server arises by virtue of computer programs running on the respective computers and having a client-server relationship to each other.

Although a few variations have been described in detail above, other modifications are possible. For example, the logic flow depicted in the accompanying figures and described herein do not require the particular order shown, or sequential order, to achieve desirable results. Other embodiments may be within the scope of the following claims.

## Claims

1. A mobile device user networking system (140), comprising:
a database (180) accessible via a communications network (170), the database storing information which is provided by one or more users of the communications network; and
an information engine (185) configured to:
receive a query from a mobile device (150) via the communications network,
query the database (180) for relevant matches to the query to generate a list of returned answers,
determine a context of the query,
compare the query and the context with the information in the database (180) to generate a contextual result,
identify one or more users associated with the contextual result, and
generate a directory of identifiers representing the identified one or more users;
wherein, to generate the contextual result, the information engine (185) is further configured to categorize and index the query as well as the list of returned answers with respect to the context.

2. A system in accordance with claim 1, wherein the communications network (170) includes a wireless communications network.

3. A system in accordance with claim 1, wherein the communications network (170) includes a messaging system for sending messages to and from the mobile device.

4. A system in accordance with claim 2, wherein the identifiers include a telephone number of a mobile device associated with at least one of the identified one or more users.

5. A system in accordance with claim 3, wherein the identifiers include a messaging address of a mobile device associated with at least one of the identified one or more users.

6. A system in accordance with claim 1, wherein the information engine (185) is comprised of a server running an application program.

7. A mobile device user networking method, comprising:
receiving a query from a mobile device via a communications network (step 206);
querying a database relevant matches to the query in order to obtain a list of returned answers;
determining a context of the query (step 208);
comparing the query and the context with information in the database that is accessible via the communications network to generate a contextual result, wherein the database stores information which is provided by one or more users of the communications network (step 210);
categorizing and indexing the query as well as the list of returned answers with respect to the context, in order to generate the contextual result;
identifying one or more users associated with the contextual result (step 212); and
generating a directory of identifiers representing the identified one or more users (step 216).

8. A method in accordance with claim 7, further comprising transmitting the directory of identifiers to the mobile device (150) via the communications network (170).

9. A method in accordance with claim 8, further comprising notifying, in a message transmitted via the communications network (170), at least one of the identified one or more users of at least one of the query and the context.

10. A method in accordance with claim 9, wherein the message includes an identification number of the mobile device.

11. A method in accordance with claim 7, wherein the communications network (170) includes a wireless communications network for transmitting voice and/or data signals.

12. A method in accordance with claim 7, wherein the communications network (170) includes a messaging system for transmitting messages.

13. A method in accordance with claim 11, wherein the identifiers include a telephone number of a mobile device associated with at least one of the identified one or more users.

14. A method in accordance with claim 12, wherein the identifiers include a messaging address of a mobile device associated with at least one of the identified one or more users.

## Patentansprüche

1. Netzwerksystem (140) für Nutzer mobiler Geräte, wobei das Netzwerksystem (140) die folgenden Elemente umfasst:
eine Datenbank (180), die über ein Kommunikationsnetzwerk (170) zugreifbar ist, wobei die Datenbank Informationen speichert, die von einem oder mehreren Nutzern des Kommunikationsnetzwerks zur Verfügung gestellt werden; und
eine Informationsmaschine (185), die konfiguriert ist zum:
Empfangen einer Abfrage von einem mobilen Gerät (150) über das Kommunikationsnetzwerk;
Abfragen der Datenbank (180) nach relevanten Übereinstimmungen zu der Abfrage, um eine Liste von zurückgegebenen Antworten zu erzeugen;
Bestimmen eines Kontextes der Abfrage;
Vergleichen der Abfrage und des Kontextes mit den Informationen in der Datenbank (180), um ein kontextabhängiges Ergebnis zu erzeugen;
Identifizieren eines oder mehrerer Nutzer, die mit dem kontextabhängigen Ergebnis assoziiert sind; und
Erzeugen eines Verzeichnisses von Bezeichnern, die die identifizierten ein oder mehreren Nutzer repräsentieren;
wobei zum Erzeugen des kontextabhängigen Ergebnisses die Informationsmaschine (185) des Weiteren konfiguriert ist, die Abfrage sowie die Liste von zurückgegebenen Antworten in Bezug auf den Kontext zu kategorisieren und zu indizieren.

2. Netzwerksystem gemäß Anspruch 1, wobei das Kommunikationsnetzwerk (170) ein drahtloses Kommunikationsnetzwerk beinhaltet.

3. Netzwerksystem nach Anspruch 1, wobei das Kommunikationsnetzwerk (170) ein Nachrichtensystem zum Senden von Nachrichten zu und von dem mobilen Gerät beinhaltet.

4. Netzwerksystem nach Anspruch 2, wobei die Bezeichner eine Telefonnummer eines mobilen Geräts, das mit zumindest einem der identifizierten ein oder mehreren Nutzer assoziiert ist, beinhalten.

5. Netzwerksystem nach Anspruch 3, wobei die Bezeichner eine Nachrichtenadresse eines mobilen Geräts, das mit zumindest einem der identifizierten ein oder mehreren Nutzer assoziiert ist, beinhalten.

6. Netzwerksystem gemäß Anspruch 1, wobei die Informationsmaschine (185) von einem Server, der ein Anwendungsprogramm ausführt, gebildet wird.

7. Netzwerkverfahren für Nutzer mobiler Geräte, wobei das Netzwerkverfahren die folgenden Schritte umfasst:
Empfangen einer Abfrage von einem mobilen Gerät über ein Kommunikationsnetzwerk (Schritt 206);
Abfragen einer Datenbank nach relevanten Übereinstimmungen zu der Abfrage, um eine Liste von zurückgegebenen Antworten zu erhalten;
Bestimmen eines Kontextes der Abfrage (Schritt 208);
Vergleichen der Abfrage und des Kontextes mit Informationen in der Datenbank, die über das Kommunikationsnetzwerk zugreifbar ist, um ein kontextabhängiges Ergebnis zu erzeugen, wobei die Datenbank Informationen speichert, die von einem oder mehreren Nutzern des Kommunikationsnetzwerks zur Verfügung gestellt werden (Schritt 210);
Kategorisieren und Indizieren der Abfrage sowie der Liste von zurückgegebenen Antworten in Bezug auf den Kontext, um das kontextabhängige Ergebnis zu erzeugen;
Identifizieren eines oder mehrerer Nutzer, die mit dem kontextabhängigen Ergebnis assoziiert sind (Schritt 212); und
Erzeugen eines Verzeichnisses von Bezeichnern, die die identifizierten ein oder mehreren Nutzer repräsentieren (Schritt 216).

8. Netzwerkverfahren nach Anspruch 7, wobei das Netzwerkverfahren des Weiteren ein Übertragen des Verzeichnisses von Bezeichnern zu dem mobilen Gerät (150) über das Kommunikationsnetzwerk (170) umfasst.

9. Netzwerkverfahren nach Anspruch 8, wobei das Netzwerkverfahren des Weiteren ein Benachrichtigen zumindest eines der identifizierten ein oder mehreren Nutzer über zumindest eines der Abfrage und des Kontextes in einer Nachricht, die über das Kommunikationsnetzwerk (170) übertragen wird, umfasst.

10. Netzwerkverfahren nach Anspruch 9, wobei die Nachricht eine Identifikationsnummer des mobilen Gerätes beinhaltet.

11. Netzwerkverfahren nach Anspruch 7, wobei das Kommunikationsnetzwerk (170) ein drahtloses Kommunikationsnetzwerk zum Übertragen von Sprache und/oder Datensignalen beinhaltet.

12. Netzwerkverfahren nach Anspruch 7, wobei das Kommunikationsnetzwerk (170) ein Nachrichtensystem zum Übertragen von Nachrichten beinhaltet.

13. Netzwerkverfahren nach Anspruch 11, wobei die Bezeichner eine Telefonnummer eines mobilen Geräts, das mit zumindest einem der identifizierten ein oder mehreren Nutzer assoziiert ist, beinhalten.

14. Netzwerkverfahren nach Anspruch 12, wobei die Bezeichner eine Nachrichtenadresse eines mobilen Gerätes, das mit zumindest einem der identifizierten ein oder mehreren Nutzer assoziiert ist, beinhalten.

## Revendications

1. Système de réseautage (140) pour utilisateurs de dispositif mobile, comprenant :
une base de données (180) accessible par l'intermédiaire d'un réseau de communication (170), la base de données stockant des informations qui sont fournies par un ou des utilisateurs du réseau de communication ; et
un moteur d'informations (185) configuré pour :
recevoir une interrogation à partir d'un dispositif mobile (150) par l'intermédiaire du réseau de communication,
interroger la base de données (180) sur des correspondances pertinentes relativement à l'interrogation afin de générer une liste de réponses renvoyées,
déterminer un contexte de l'interrogation,
comparer l'interrogation et le contexte aux informations de la base de données (180) pour produire un résultat contextuel,
identifier un ou des utilisateurs associés au résultat contextuel, et
produire un répertoire d'identifiants représentant le ou les utilisateurs identifiés ;
dans lequel, pour produire le résultat contextuel, le moteur d'informations (185) est en outre configuré pour catégoriser et indexer l'interrogation ainsi que la liste de réponses renvoyées en lien avec le contexte.

2. Système selon la revendication 1, dans lequel le réseau de communication (170) comprend un réseau de communication sans fil.

3. Système selon la revendication 1, dans lequel le réseau de communication (170) comprend un système de messagerie pour envoyer des messages au et à partir du dispositif mobile.

4. Système selon la revendication 2, dans lequel les identifiants comprennent un numéro de téléphone d'un dispositif mobile associé à au moins un du ou des utilisateurs identifiés.

5. Système selon la revendication 3, dans lequel les identifiants comprennent une adresse de messagerie d'un dispositif mobile associé à au moins un du ou des utilisateurs identifiés.

6. Système selon la revendication 1, dans lequel le moteur d'informations (185) se compose d'un serveur exécutant un programme d'application.

7. Procédé de réseautage d'utilisateurs de dispositifs mobiles, comprenant les étapes consistant à :
recevoir une interrogation à partir d'un dispositif mobile par l'intermédiaire d'un réseau de communication (étape 206) ;
interroger une base de données sur des correspondances pertinentes pour l'interrogation afin d'obtenir une liste de réponses renvoyées ;
déterminer un contexte de l'interrogation (étape 208) ;
comparer l'interrogation et le contexte à des informations de la base de données qui est accessible par l'intermédiaire du réseau de communication de manière à générer un résultat contextuel, dans lequel la base de données stocke des informations qui sont fournies par un ou des utilisateurs du réseau de communication (étape 210) ;
catégoriser et indexer l'interrogation ainsi que la liste de réponses renvoyées par rapport au contexte, afin de générer le résultat contextuel ;
identifier un ou des utilisateurs associés au résultat contextuel (étape 212) ; et
générer un répertoire d'identifiants représentant le ou les utilisateurs identifiés (étape 216).

8. Procédé selon la revendication 7, comprenant en outre l'étape consistant à transmettre le répertoire d'identifiants au dispositif mobile (150) par l'intermédiaire du réseau de communication (170).

9. Procédé selon la revendication 8, comprenant en outre l'étape consistant à informer, dans un message transmis par l'intermédiaire du réseau de communication (170), au moins un du ou des multiples utilisateurs identifiés d'au moins l'un de l'interrogation et du contexte.

10. Procédé selon la revendication 9, dans lequel le message comprend un numéro d'identification du dispositif mobile.

11. Procédé selon la revendication 7, dans lequel le réseau de communication (170) comprend un réseau de communication sans fil pour transmettre des signaux vocaux et/ou de données.

12. Procédé selon la revendication 7, dans lequel le réseau de communication (170) comprend un système de messagerie pour transmettre des messages.

13. Procédé selon la revendication 11, dans lequel les identifiants comprennent un numéro de téléphone d'un dispositif mobile associé à au moins un du ou des utilisateurs identifiés.

14. Procédé selon la revendication 12, dans lequel les identifiants comprennent une adresse de messagerie d'un dispositif mobile associé à au moins un du ou des utilisateurs identifiés.
